# FASCICULE DE BREVET EUROPEEN

(11) **EP 3 463 587 B1**
(45) Date de publication et mention de la délivrance du brevet: **07.01.2026**
(21) Numéro de dépôt: 17733491.9
(22) Date de dépôt: 24.05.2017
(51) Int. Cl.: A61Q 19/00, A61Q 19/08, A61K 8/96, A61K 9/00, A61K 47/10, A61K 47/22, A61K 9/08, A61K 47/36, A61K 47/46, A61K 8/04, A61K 8/06

(54) **COMPOSITIONS COSMÉTIQUE COMPRENANT DE L'EAU DE ZAMZAM**
KOSMETISCHE ZUSAMMENSETZUNGEN ENTHALTEND ZAMZAM-WASSER
COSMETIC COMPOSITIONS COMPRISING ZAMZAM WATER

(30) Priorité: 24.05.2016 FR 1670259
(43) Date de publication de la demande: 10.04.2019
(73) Titulaire: FL Nova, 92130 Issy-les-Moulineaux (FR)
(72) Inventeur: DUMAS, Françoise, F-92350 Le Plessis Robinson (FR); QUTISHAT, Léon Laith, F-91300 Massy (FR)
(74) Mandataire: Touroude, Magali Linda
(86) Numéro de dépôt international: PCT/FR2017/000106
(87) Numéro de publication internationale: WO 2017/203111

(56) Documents cités:
- EP-A1- 1 166 762
- WO-A1-2017/050830
- US-B1- 8 852 651
- SHOMAR BASEM ED - SONG CHUL H ET AL: "Zamzam water: Concentration of trace elements and other characteristics", CHEMOSPHERE, vol. 86, no. 6, 3 December 2011 (2011-12-03), pages 600 - 605, XP028886632, ISSN: 0045-6535, DOI: 10.1016/J.CHEMOSPHERE.2011.10.025

## Description

### DOMAINE TECHNIQUE

La présente invention concerne le domaine du soin de la peau et vise notamment à améliorer l'hydratation de la peau. Elle concerne en particulier l'utilisation de l'eau de source de Zamzam dans une composition cosmétique et/ou dermatologique pour son effet anti-âge et hydratant de la peau, ainsi que pour empêcher le relâchement cutané.

### ETAT DE LA TECHNIQUE ANTERIEURE

Les formules cosmétiques comme les crèmes eau dans huile (E/H) ou huile dans eau (H/E), pommade (H/E), lotion, gel, se constituent de phase aqueuse, et/(ou sans) un émulsifiant, et/(ou sans) phase huileuse et/(ou sans) gélifiant ou les aérosols. Les formules cosmétiques ont pour objectif d'hydrater, de nettoyer, d'améliorer le toucher et l'apparence de la peau et la pénétration d'éventuels principes actifs.

La peau est l'organe le plus grand du corps humain et représente 10% de la masse corporelle. Elle protège le corps contre les facteurs exogènes (pollution, soleil, froid), contre la perte d'eau endogène et contre les microbes, elle aide à réguler la température du corps et elle permet les sensations du toucher, de la chaleur et du froid.

La peau est constituée de trois compartiments : l'hypoderme, le derme et l'épiderme. L'épiderme est un tissu épithélial de revêtement semi-perméable. Ce dernier est la couche la plus externe et la plus fine de la peau, très résistante, kératinisée et non vascularisée. Il est divisé lui-même en cinq couches. 1- la couche cornée (SC pour *stratum corneum*), composée de cornéocytes. 2- la couche claire (SL pour *Stratum lucidum*), qui correspond à une phase de transition entre la couche granuleuse et la couche cornée. 3- la couche granuleuse (SG *pour Stratum granulosum*) où commence la kératinisation des kératinocytes (qui évoluent en cornéocytes). 4- la couche épineuse (SS pour *Stratum spinosum*) ou corps muqueux de malpighie. 5- la couche basale (SB pour *Stratum basale*), la plus profonde de l'épiderme. Elle assure la régénération continue de la peau par division cellulaire : les cellules produites migrent progressivement vers les couches supérieures en subissant diverses mutations.

Le SC a une structure globale relativement simple avec des cellules appelées cornéocytes reliées par des desmosomes, des zones d'adhérences des cellules ou zones de connexion. L'ensemble est imprégné d'une matrice de lipides qui remplit l'espace intercellulaire. Ces couches de cellules avec leur connections représentent entre 75 et 80% du volume du SC. Les 20-25% restants correspondent à la matrice lipidique. L'épaisseur totale du SC est évaluée à 15-20µm.

Dans une situation physiopathologique accompagnée par un défaut dans l'adsorption de l'eau, et de la fonction de barrière (âge, statut hormonal, maladies de la peau). Dans ce cas, la capacité du SC d'initier une réponse adaptative à une perturbation est réduite, voire inexistante, et doit donc être compensée. La sécheresse cutanée constitue un phénomène commun dans différents dysfonctionnements physiopathologiques. L'hydratation cutanée possède une place dominante en cosmétique et en dermatologie. Les signes de la peau sèche sont : une peau qui est souvent froissée, dure et rugueuse, qui a tendance à desquamer. La peau déshydratée est une peau qui a perdu son élasticité. En outre, une peau sèche, sauf dans le cas de maladies, est souvent synonyme de vieillissement de la peau. D'autres facteurs de la déshydratation cutanée sont les effets climatiques et les expositions solaires.

Dans le derme, une partie de l'eau est présente sous forme de gel, fixé à de nombreuses macromolécules hydrophiles. En dépit de la grande capacité de ces molécules à capter l'eau, une partie de l'eau reste mobile et diffuse vers l'épiderme qui sert de première barrière de protection à l'environnement extérieur. Trois types d'eau sont présentes dans la peau ; l'eau non liée (ENL), l'eau partiellement liée (EPL) et l'eau totalement liée (ETL). L'eau se déplace entre ces trois formes pour établir un équilibre. La majorité de l'eau dermique est totalement liée. De ce fait, entre le derme fortement hydraté et l'épiderme, un gradient décroissant existe et devient plus évident entre les couches épidermiques les plus profondes et le SC. En particulier, alors que toutes les couches vivantes de l'épiderme, le SB, le SS et le SG présentent un niveau d'hydratation élevé, de l'ordre de 70%, à poids égal, une chute brutale de l'hydratation suit la transition entre SG et SC. A cet endroit, le niveau d'hydratation passe à 30% et retombe à 15% environ dans les couches les plus superficielles. Le mouvement d'eau contribue au bon fonctionnement du SC par un approvisionnement continu en eau.

La peau est recouverte d'une couche lipidique (nourrie notamment par le sébum) exerçant un rôle de barrière, à la fois contre les agressions extérieures et contre les pertes en eau. Si cette barrière lipidique est affaiblie, l'évaporation de l'eau (ou perte d'eau trans-épidermique) est plus importante. Ainsi, le renforcement de la barrière lipidique est important pour l'hydratation de la peau et pour éviter le vieillissement. La déformation de la barrière cutanée est une des manifestations de la dermatite atopique ou secondaire. Dans des conditions physiologiques normales, les céramides avec les acides gras et le cholestérol forment un réseau organisé de façon optimale qui assure un flux d'eau trans-épidermique modéré. D'une manière générale, les modifications quantitatives et/ou qualitatives de n'importe quel lipide de ces trois classes se traduisent par des anomalies de la barrière cutanée qui se caractérise par une augmentation de la Perte Insensible en Eau (PIE) ainsi que des modifications organisationnelles supra-moléculaires de la matrice lipidique extracellulaire.

En général, le stress de séchage biomécanique du SC est dû aux contributions de la perte à la fois de l'ENL et l'EPL, et elle dépend principalement de la fraction d'ENL. Cette fraction est située dans l'intervalle des composants de la couche cornée et agit comme un lubrifiant. L'ENL permet aux molécules du SC de se déplacer librement les unes par rapport aux autres et ceci augmente considérablement la souplesse du tissu.

L'interaction eau - lipides est produite probablement avec les têtes polaires des lipides. Ainsi, elle pourrait conduire à une modification de la conformation des chaînes d'hydrocarbure et notamment leur organisation supramoléculaire.

Deux types principaux d'agents hydratants existent classiquement. Les agents occlusifs ou filmogènes s'opposent à la déshydratation en formant un film gras à la surface de la peau qui limite la PIE. Ce sont tous des lipides ou des hydrocarbures. Les plus couramment utilisés sont la vaseline, l'huile de paraffine, le perhydrosqualène, les huiles de silicone, les huiles animales et végétales, les alcools gras, les cires (beurre de karité, lanoline, etc...). Ces produits ne sont pas utilisés seuls mais sous forme d'émulsions E/H. Les autres agents hydratants sont les humectants très hygroscopiques qui ont une forte affinité pour l'eau. Ils comprennent les facteurs naturels d'hydratation de la peau ou NMFs (pour Natural Moisturizing Factors) ou des complexes constitués de ces éléments les plus actifs, notamment le lactate de sodium, les sels de sodium de l'acide pyrrolidone carboxylique et l'urée. Les polyols, tels que l'éthylène glycol, le glycérol ou le propylène glycol sont également utilisés en tant qu'humectants. A ces deux types majeurs s'ajoutent les modulateurs de la barrière cutanée. Ces molécules changent la conformation et l'organisation des lipides du SC en leur conférant une meilleure compacité. La structure des protéines ainsi que la compacité de la barrière lipidique du SC sont directement liés à la variation de l'EPL (R. Vyumvuhore, Thèse de doctorat, Université Paris Sud, 2013).

L'apport d'actifs hydratants et la limitation des pertes insensibles en eau sont les deux axes principaux pour maintenir et renforcer cette barrière cutanée et par conséquent le bon état général de la peau. De nouveaux actifs hydratants et/ou capables de diminuer les pertes insensibles en eau présentent donc ainsi un grand intérêt au niveau cosmétique et pharmaceutique, notamment dermatologique.

Les conséquences d'une mauvaise hydratation de la peau, souvent associées aux altérations liées à l'âge telles que les rides et ridules, rougeurs, d'origine multifactorielle, sont une cause de plus en plus fréquente de consultations auprès de centres de soins esthétiques ou de cabinets dermatologiques. Elles concernent presque chaque individu, et leur fréquence est maximale à partir de 40 ans, et notamment au-delà de 60 ans.

Il existe donc un réel besoin d'être en mesure de prévenir, de réduire ou de traiter ce manque d'hydratation de la peau, et donc ces altérations de l'aspect de la peau liées au manque d'hydratation, et également souvent liées à l'âge, en particulier de prévenir l'apparition des rides et/ou ridules de la peau, et/ou diminue les rides et/ou ridules d'une peau ridée, et/ou prévenir le relâchement cutané, et/ou diminuer le relâchement cutané, et ou prévenir et/ou réduire les rougeurs de la peau.

Le document EP 1 166 762 A1 décrit une composition cosmétique comprenant de l'eau minérale utilisée pour l'hydratation de la peau. Dans ce document, l'eau utilisée présente des concentrations en sodium comprises entre 1-10 mg/M, 0,1-5 mg/L pour le potassium et entre 30-150 mg/L pour le calcium.

La Demanderesse a mis en évidence de manière surprenante et inattendue que l'utilisation d'une eau de source ayant une concentration en sodium comprise entre environ 85 mg/L et environ 150 mg/L et une concentration en potassium comprise entre environ 35 et environ 60 mg/L, en particulier l'eau de la source Zamzam, pour la fabrication d'une composition cosmétique, permettait dans le cadre d'un application topique sur la peau de sujets d'observer une augmentation significative de l'hydratation.

### EXPOSE DE L'INVENTION

Ainsi un premier objet de l'invention concerne une utilisation cosmétique non-thérapeutique pour hydrater la peau d'une composition cosmétique destinée à une application topique, sur la peau, ladite composition comprenant de l'eau de source comprenant :- du sodium dans une concentration comprise entre 85 mg/L et 150 mg/L, et du potassium dans une concentration comprise entre 35 et 60 mg/L ;- du calcium, dans une concentration comprise entre 50 et 230 mg/L ; ladite eau de source ayant une minéralisation comprise entre 650 mg/L et 1100 mg/l, de préférence entre 780 et 1000 mg/L.

De manière préférée selon l'invention, la composition minérale de ladite eau de source comprend en outre :
- Du calcium, dans une concentration comprise entre 53 et 115 mg/L et de préférence en outre
- Du magnésium dans une concentration comprise entre 10 et 80 mg/L et/ou
- Du bicarbonate dans une concentration comprise entre 150 et 195 mg/L et/ou
- Du Chlorure dans une concentration comprise entre 160 mg/L et 260 ml/L et/ou
- Des fluorures dans une concentration comprise entre à 0,50 et 0,8 mg/L et/ou
- Du nitrate dans une concentration comprise entre à 25 mg/L et 145 mg/L et/ou
- Du sulfate dans une concentration comprise entre à 120 et 190 mg/L

Ladite eau de source a une minéralisation comprise entre 650 mg/L et 1100 mg/l, de préférence entre 780 et 1000 mg/L.

Par « minéralisation » on entend désigner selon la présente invention la somme des concentrations en anions et en cations présents dans l'eau.

Par eau de source, on entend désigner selon la présente invention une eau d'origine naturelle provenant d'une nappe ou d'un gisement souterrain, qui lui confère des caractéristiques microbiologistes saines, et lui permet d'être à l'abri de tout risque de pollution. Selon la législation française, l'eau de source selon la présente invention peut être nommée également eau minérale (articles R1321-84 à 90 du Code de la santé publique).

De manière particulièrement préférée selon l'invention, ladite eau de source est l'eau de la source de Zamzam.

L'eau de la source de Zamzam, appelée aussi « eau de Zamzam », provient d'une source située à la Mecque, en Arabie Saoudite. La source de l'eau de Zamzam peut également être désignée « source de Zemzem », ou « Bir Ismaël ».

Ladite eau de la source Zamzam possède une teneur en minéraux particulière, qui peut varier au cours du temps, suivant l'année et le procédé utilisé (voir par exemple Basem Shomar « Zamzam water : concentration of trace elements and other characteristics, Chemosphere 86 (2012) 600-605, Al Zuhair N. et Khounganian R. A comparative study between the chemical composition of potable water and Zamzam water in Saudi Arabia. (2006), El-Zaiat, S.Y. Inherent optical properties of Zamzam water in the visible spectrum: dispersion analysis. The Arabian Journal for Science and Engineering 2007, 32 (2A), 171-180 et Alfadul, S.M.; Khan, M.A. Water quality of bottled water in the kingdom of Saudi Arabia: A comparative study with Riyadh municipal and Zamzam water. Journal of Environmental Science and Health Part A 2011, 46 (13), 1519-1528, Doha Al Nouri, Badriah Al Abdulkarim, Shaista Arzoo, Zubaida Abdel Nabi Bakeet « Quality Characteristics of Commonly Consumed Drinking Water in Riyadh and Effect of Domestic Treatments on Its Chemical Constituents » Journal of Food and Nutrition Research. 2014), et qui comprend :
- Du sodium dans une concentration comprise entre 85 mg/L et 150 mg/L de préférence entre 90 et 140 mg/L, de préférence ente 90 et 133 mg/L, et
- Du potassium dans une concentration comprise entre 35 et 60 mg/L, de préférence entre 40 et 50 mg/L, et de préférence en outre
- Du calcium, dans une concentration comprise entre 50 et 230 mg/L, de préférence entre 53 et 115 mg/L et
- Du magnésium dans une concentration comprise entre 10 et 80 mg/L et/ou
- Du bicarbonate dans une concentration comprise entre 150 et 195 mg/L et/ou
- Du Chlorure dans une concentration comprise entre 160 mg/L et 260 ml/L et/ou
- Des fluorures dans une concentration comprise entre à 0,50 et 0,8 mg/L et/ou
- Du nitrate dans une concentration comprise entre à 25 mg/L et 145 mg/L et/ou
- Du sulfate dans une concentration comprise entre à 120 et 190 mg/L
- Un pH compris entre 6,5 et 8,5
- De préférence une minéralisation comprise entre 650 mg/L et 1100 mg/l, de préférence entre 780 et 1000 mg/L, et

De manière préférée, l'eau de Zamzam selon la présente invention est l'eau de Zamzam commercialisée embouteillée par les autorités saoudiennes dont la composition est la suivante :
- Du sodium dans une concentration comprise entre environ 91 mg/L et environ 93 mg/L et
- Du potassium dans une concentration comprise entre environ 42 et environ 44 mg/L et
- Du calcium, dans une concentration comprise entre environ 55 et environ 57 mg/L et

A titre de comparaison est présenté ci-après la minéralisation d'autres eaux de sources disponibles sur le marché français :

**Tableau 2 : Concentration minérale d'eau de sources commercialisées (mg/L)**

| Composition | Chantereine | Volvic | Mont calm | Contrex |
|---|---|---|---|---|
| Calcium | 119 | 9,9 | 3 | 467 |
| **Sodium** | **7** | **9,4** | **1,5** | **7** |
| Magnésium | 28 | 6,1 | 0,6 | 84 |
| Bicarbonate | 430 | 65,3 | | 377 |
| Sulfates | 5,2 | 6,9 | 8,7 | 1192 |
| Nitrates | 0 | 6,3 | 0,9 | - |
| **Potassium** | **2** | **5,7** | **0,4** | **3** |

L'utilisation de cette l'eau de Zamzam dans une composition pharmaceutique, nutraceutique ou alimentaire a été décrite (US8852651), pour le traitement ou en prophylactique des troubles du système immunitaire, de l'anxiété, des dysfonctions ou déficiences de la mémoire, du manque de concentration, du bien-être émotionnel diminué, de la mauvaise humeur, des dysfonctionnements sexuels, de l'impuissance, et du manque d'appétit.

En plus dudit effet thérapeutique ou prophylactique, l'eau de la source de Zamzam a également été caractérisée comme un excellent inhibiteur de corrosion de l'acier (Elshami , Bonnet S et Khelidj A. Zamzam Water as Corrosion Inhibitor for Steel Rebarin Rainwater and Simulated Acid Rain. International Journal of Chemical, Molecular, Nuclear, Materials and Metallurgical Engineering Vol:8, No:9, 2014) dans les eaux de pluie ou les eaux de pluies simulées.

De manière préférée selon l'invention, ladite eau de source est présente à hauteur de concentrations allant de 0,01% à environ 90% en poids de la composition, de préférence d'environ 0,1% à environ 75% en poids de la composition, plus préférablement d'environ 1,0% à environ 50% en poids de la composition et plus préférablement encore d'environ 1,0% à environ 25% en poids de la composition.

La composition mise en œuvre dans la présente invention peut se présenter sous toutes les formes galéniques normalement utilisées dans les domaines cosmétique et dermatologique et compatibles avec les caractéristiques de ladite composition.

Les formulations selon l'invention seront préparés selon les méthodes classiques bien connues de l'Homme du métier telles que celles décrites dans "Pharmaceutical Handbook Remington», Mack Publishing Co., NY, Etats-Unis, avec des excipients appropriés.

La composition peut être employée selon toutes les façons envisageables par l'homme du métier. La composition peut être plus ou moins fluide et avoir l'aspect d'une crème blanche ou colorée, d'une pommade, d'un lait, d'une lotion, d'un sérum, d'une pâte, ou d'une mousse. Elle peut éventuellement être appliquée sur la peau sous forme d'aérosol. Elle peut également se présenter sous forme solide, et par exemple sous forme de stick.

De manière préférée la composition cosmétique utilisée selon l'invention est sous la forme d'une crème blanche ou colorée, en particulier crème de nettoyage, de protection, de traitement ou de soin pour le visage, pour les mains, pour les pieds, pour les grands plis anatomiques ou pour le corps, (par exemple crème de jour, crème de nuit, crème démaquillante, crème de fond de teint, crème anti-solaire), de fond de teint fluide, d'une pommade, d'un lait, en particulier lait anti-solaire, d'une lotion, gel ou mousse pour le soin de la peau, comme lotion de nettoyage, lotion anti-solaire, lotion de bronzage artificiel, d'un sérum, d'une pâte, de gel-crèmes, d'émulsions, de solutions aqueuses, de gels, de pommades, d'aérosols, de stick ou de poudre pressé, de composition pour le bain, composition désodorisante, comprenant un agent bactéricide, gel ou lotion après-rasage, crème épilatoire, composition contre les piqûres d'insectes.

De manière préférée, la composition cosmétique utilisée selon l'invention comprend en outre des excipients et des actifs compatibles entre eux formant un milieu acceptable, lesdits actifs pouvant être choisi parmi : les huiles végétales, les huiles minérales, glycérines, les huiles essentielles, les minéraux, les acides aminés et analogues, les vitamines et ses dérivés, et les acides gras et les acides gras éthoxylés et leurs dérivés, les extraits végétaux, l'urée et les dérivés de silices, les dérivés de polyols, l'acide lactique, les acides de fruits et des actifs qui ont un effet hydratant, émollient ou effet antiâge.

Par milieu acceptable sur le plan cosmétique, on comprend un milieu compatible avec les matières kératiniques d'êtres humains comme la peau, les muqueuses, les ongles, le cuir chevelu et/ou les cheveux.

La composition mise en œuvre dans la présente invention est destinée à une application topique, sur la peau, telle que défnie dans les revendications. Un milieu acceptable sur le plan cosmétique selon la présente invention peut être de la glycérine dans des quantités comprises entre environ 0,10 et environ 20%.

Les compositions peuvent également comprendre tous les constituants usuellement employés dans l'application envisagée. En particulier, de façon avantageuse, on pourra utiliser dans les compositions, en complément de l'association décrite précédemment, au moins un ingrédient et/ou un actif additionnel de soin de la peau, en particulier de la peau grasse.

On pourra notamment associer les actifs et ingrédients additionnels décrits dans la demande de brevet WO 2004/105736, parmi lesquels les agents dépigmentants, les conservateurs, les agents anti-transpirants, les agents séborégulateurs, les chélateurs de métaux, les protéines hydrolysées, les antioxydants, les vitamines, les agents apaisants ou anti-irritants, les agents hydratants, les extraits végétaux, les adjuvants cosmétiques, et leurs mélanges.

Bien entendu, l'homme du métier veillera à choisir ce ou ces éventuels composés complémentaires, et/ou leur quantité, de manière telle que les propriétés avantageuses des composés selon l'invention ne soient pas, ou substantiellement pas, altérées par l'adjonction envisagée.

La composition mise en œuvre dans la présente invention étant destinée à une utilisation topique sur la peau, le véhicule ou milieu doit être acceptable sur le plan cosmétique ou dermatologique, qui ne génère pas de picotement, tiraillement ou rougeur inacceptable pour l'utilisateur.

Selon un mode de réalisation, la composition cosmétique et/ou dermatologique selon l'invention est caractérisée en ce qu'elle comprend de l'eau de source de Zamzam, de l'isopropyl palmitate, de l'alcool cétéarylique & Polysorbate 60, de la glycérine, de la gomme xanthane, de l'acétate de tocophérol, et du 5-Chloro-2-méthylisothiazolin-3-one.

Cette invention se rapporte à l'utilisation de la composition cosmétique selon l'invention pour hydrater la peau, de préférence en renforçant la barrière lipidique de la peau. Cette utilisation est particulièrement indiquée pour le traitement des peaux sèches.

Au sens de la présente invention, on entend par « hydrater la peau », augmenter la teneur en eau de la peau et/ou des muqueuses, c'est à dire la quantité d'eau contenue dans les épithélia, en particulier l'épiderme et/ou l'épithélium des muqueuses. Cette teneur traduit ainsi l'état d'hydratation. Il existe différentes méthodes de mesure de la teneur en eau de la peau et/ou des muqueuses dans les connaissances générales de l'homme du métier, notamment in vivo et en particulier via le cornéomètre, et/ou par la mesure de la desquamation via le cornéofix, ou in vitro, notamment via la mesure de la conductivité diélectrique.

Ces propriétés hydratantes de la composition selon l'invention sont notamment apportées de façon durable à la peau après une application répétée, c'est à dire qu'elles persistent plusieurs jours et/ou une ou plusieurs semaines après application, même si la peau, n'est plus en contact avec la composition selon l'invention

De manière préférée, la composition cosmétique selon l'invention est utilisée pour prévenir et/ou réduire le relâchement cutané et pour son effet anti-âge sur la peau et/ou pour son effet anti-rougeurs sur la peau.

Par effet anti-âge, on entend selon la présente invention prévenir l'apparition des rides et/ou ridules de la peau, et/ou diminue les rides et/ou ridules d'une peau ridée et/ou diminuer ou réduire les rougeurs.

Selon un autre aspect cette invention se rapporte également à l'utilisation de la composition cosmétique selon l'invention pour prévenir ou réduire les rougeurs de la peau. L'apparition de rougeurs sur la peau peut être lié à l'âge et donc se confondre avec un effet anti-âge, mais peut également être lié à d'autres facteurs.

### LEGENDE DES FIGURES

**Figure 1** **:** Illustration des zones d'application
**Figure 2** **:** Extrait de spectre Raman à 5 µm de profondeur (bénévole 02).
**Figure 3** : Histogrammes 1 représentant l'eau non liée (ENL) à trois profondeurs et à T0 (jour 1) et T4 (jour 28) du volontaire 03. (Témoin: zone témoin, Droite: zone de l'avant-bras droit, Gauche: zone de l'avant bras gauche, voir Figure 1/13).
**Figure 4** **:** Histogrammes 2 représentant l'eau partiellement liée (EPL) à trois profondeurs et à T0 (jour 1) et T4 (jour 28) du volontaire 03. (Témoin: zone témoin, Droite: zone de l'avant-bras droit, Gauche: zone de l'avant bras gauche, voir Figure 1/13).
**Figure 5****:** Histogrammes 3 représentant l'eau étroitement liée (EEL) à trois profondeurs et à T0 (jour 1) et T4 (jour 28) du volontaire 03. (Témoin: zone témoin, Droite: zone de l'avant-bras droit, Gauche: zone de l'avant bras gauche, voir Figure 1/13).
**Figure 6** **:** Histogrammes 4a représentant l'eau non liée (ENL) à la profondeur de 15 µm et à T0 (jour 1) et T4 (jour 28) du volontaire 03. (Témoin: zone témoin, Droite: zone de l'avant-bras droit, Gauche: zone de l'avant bras gauche, voir Figure 1/13).
**Figure 7** **:** Histogrammes 4b représentant l'eau partiellement liée (EPL) à la profondeur de 15 µm et à T0 (jour 1) et T4 (jour 28) du volontaire 03. (Témoin: zone témoin, Droite: zone de l'avant-bras droit, Gauche: zone de l'avant bras gauche, voir Figure 1/13).
**Figure 8** **:** Histogrammes 4c représentant l'eau étroitement liée (EEL) à la profondeur de 15 µm à T0 (jour 1) et T4 (jour 28) du volontaire 03. (Témoin: zone témoin, Droite: zone de l'avant-bras droit, Gauche: zone de l'avant bras gauche, voir Figure 1/13).
**Figure 9** **:** Graphique 1 considérant le contenu global de l'eau (vOH: aire sous la courbe ASC) pour tous les bénévoles à la profondeur de 15 µm à J0, J1 (après 7 jours), J2 (à 14 jours), J3 (après 21 jours) et J4 (jour 28).
**Figure 10** **:** Graphique 2: Résultats de l'étude CORNEO (unités arbitraires) à 0 (jour 1), 1 (à 7 jours), 2 (à 14 jours), 3 (à 21 jours) et 4 (jour 28). (T: zone témoin, D: zone de l'avant-bras droit, G: zone de l'avant bras gauche, voir Figure 1/13).
**Figure 11** **:** Graphique 3 : Résultat de l'étude PIE à 0 (jour 1), 1 (à 7 jours), 2 (à 14 jours), 3 (à 21 jours) et 4 (à 28 jours). (T: zone témoin, D: zone de l'avant-bras droit, G: zone de l'avant bras gauche, voir Figure 1/13).
**Figure 12** **:** Graphique 4 représentant le ratio des ASC vasymCH2 (2885 cm-1) / vsymCH2 (2850 cm-1) pour les trois volontaires à J0 (jour 1) et J4 (jour 28), et à trois profondeurs de mesure. (T: zone témoin, BD: zone de l'avant-bras droit, BG: zone de l'avant bras gauche, voir Figure 1/13).
**Figure 13** **:** Graphique 5 : Ratios des conformations trans-gauche analysés par les rapports des ASC à 1127 + 1081 cm-1 / 1062 cm-1 à J0 (jour 1) et J4 (28 jours) à trois profondeurs. (T: zone témoin, BD: zone de l'avant-bras droit, BG: zone de l'avant bras gauche, voir Figure 1/13).

### EXEMPLE

La Demanderesse a constaté l'effet hydratant de l'eau de Zamzam sur la peau en comparant deux préparations basiques sur trois volontaires sains en double aveugle après 28 jours. Une préparation était basée sur l'eau de Zamzam commerciale embouteillée dont la composition est donnée ci-après (formule B) et l'autre préparation de référence était basée sur l'eau distillée (formule A), en gardant par ailleurs les mêmes ingrédients aux mêmes dosages pour comparer l'effet attribuable aux deux eaux différentes.

### Formule A

| | |
|---|---|
| Eau distillée | 81,6% |
| Isopropyl palmitate | 10% |
| Alcool cétéarylique & Polysorbate 60 | 7% |
| Glycérine | 1% |
| Gomme Xanthane | 0,2% |
| Acétate de tocophérol | 0,1% |
| 5-Chloro-2-méthylisothiazolin-3-one 2-Méthylisothiazolin-3-one | 0,1% |

### Formule B

| | |
|---|---|
| Eau de source Zamzam commerciale (embouteillée) | 81,6% |
| Isopropyl palmitate | 10% |
| Alcool cétéarylique & Polysorbate 60 | 7% |
| Glycérine | 1% |
| Gomme Xanthane | 0,2% |
| Acétate de tocophérol | 0,1% |
| 5-Chloro-2-méthylisothiazolin-3-one 2-Méthylisothiazolin-3-one | 0,1% |

Composition de l'Eau de Zamzam commerciale embouteillée :
- sodium : 92,1 mg/L
- Potassium : 42,54 mg/L
- Calcium : 56,1 mg/L

Nous avons mesuré l'hydratation cutanée *in vivo,* en utilisant :1- La spectroscopie de Raman confocale; 2- La mesure électrique de l'hydratation de la peau; 3- Les mesures de PIE.
1. La spectroscopie de Raman confocale a prouvé être un outil puissant, non-invasif qui peut suivre les changements des marqueurs biomoléculaires de la peau en temps réel, et de déterminer les profils de concentration de l'eau et des facteurs naturels d'hydratation ou NMFs en profondeur de la peau humaine.
2. La mesure électrique de l'hydratation de la peau consiste à mesurer son impédance électrique (EI de Electrical Impedance) définie comme la résistance électrique de la peau à un courant alternatif. Les propriétés électriques de la peau sont directement liées à la teneur en eau du SC. L'augmentation du niveau d'hydratation épidermique se traduit par des changements de propriétés électriques du SC se manifestant par une diminution de la capacitance, et de l'EI. Cette mesure est réalisée par un cornéomètre MPA 6 (Courage and Khazaka electronic GmbH, Cologne, Germany). La capacité est exprimée en unités arbitraires.
3. Les mesures de PIE (Perte Insensible en Eau) trans-épidermique ont été effectuées avec un évaporimètre TEWAmètre MPA 6 (Courage and Khazaka electronic GmbH, Cologne, Germany). La sonde a été maintenue et utilisée sur une surface stable de la peau jusqu'à ce que la PIE ait été établie.

La microscopie confocale Raman d'échantillons biologiques en permet l'analyse chimique non destructive. Cette technique est particulièrement intéressante pour l'analyse des échantillons élastiques tels que la peau. Les mesures *in vivo* en microscopie Raman ont été effectuées en utilisant une microsonde optique confocale Raman dans la gamme de 400 à 3800 cm⁻¹. La longueur d'onde d'excitation utilisée était 660 nm avec une puissance de laser sur le site de la peau de 15,0 mW. Un objectif longue distance de travail MPlanFL N 100X/NA 0,75 est couplé à un système piézo-électrique permettant des incréments de 1 µm de discontinuité. Six mesures en différents points du site de l'avant-bras ont été réalisées pour chacun des volontaires. Pour chaque analyse, une durée d'exposition de 2 secondes a été utilisée avec 2 accumulations à des profondeurs variant entre 0 et 15 µm, avec une taille de pas de 5 µm permettant d'effectuer le profilage rapide de la profondeur. Les signaux et les spectres ont été séparés en deux zones de nombre d'onde : la région 400-1800 cm⁻¹ dite « empreinte digitale », et la région 2600-3100 cm⁻¹, normalisées sur la bande d'amide I (1565-1720 cm⁻¹) et sur la bande d'étirement CH (2800-3030 cm⁻¹), respectivement.

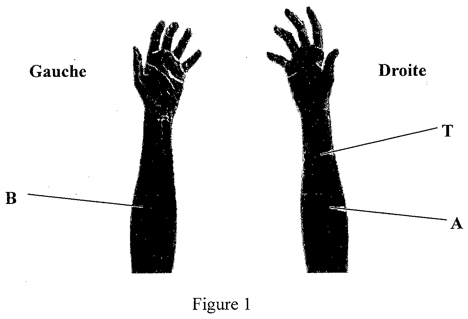

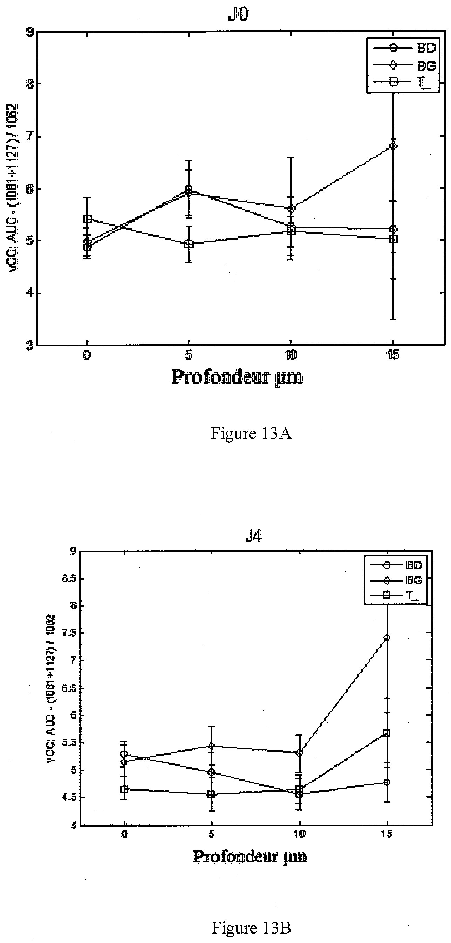

L'essai a duré 28 jours avec trois volontaires sains de sexe féminin, âgés de 23, 29 et 34 ans. Ils ont utilisé quotidiennement les deux crèmes, la formule A de référence sur la main gauche et la formule B sur la main droite (une application par jour) (Figure 1/13). Les mesures biométriques [température, PIE et EI) et la spectroscopie Raman dispersive ont été utilisées pour examiner les avant-bras respectivement de T0, au premier jour de l'expérience jusqu'au 28^{ème} jour correspondant à T4. Comme preuve du concept, nous présentons ici les résultats d'un volontaire qui a utilisé régulièrement les deux crèmes.

Les résultats montrent que les nouvelles formules générales basées sur l'eau de la source de Zamzam ont un effet hydratant par l'augmentation de l'eau dans la peau et par l'amélioration de l'intégrité de la surface lipidique.

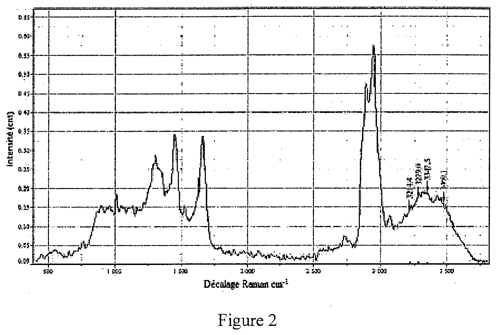

Tous les spectres de micro spectroscopie Raman ont été acquis et prétraitées selon la procédure suivante : la soustraction linéaire de base (afin de se débarrasser de la fluorescence intrinsèque de la peau), la normalisation en utilisant les deux bandes, respectivement, à 1565-1720 cm⁻¹ (qui correspond à la bande amide I) et à 2800-3030 cm⁻¹ (qui correspond à la bande d'élongation C-H). Pour le calcul du ratio, les zones intégrées sous chaque bande ont été utilisés (ASC ou aire sous la courbe). Différentes caractéristiques spectrales représentant de la fonction barrière du SC ont été utilisées pour étudier son comportement lors de l'application du protocole. (Figure 2/13). Le spectre Raman du SC est dominé par les bandes de vibration de ses protéines structurales, des lipides et l'eau des tissus. Afin d'étudier les changements biochimiques dans le SC, nous avons focalisé notre étude sur des fonctionnalités différentes des spectres.

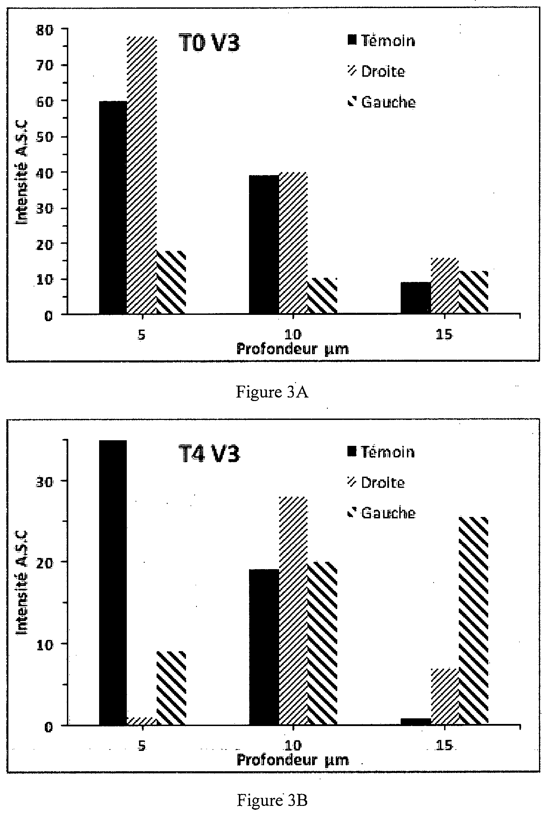

Les barres des histogrammes 1, 2 et 3 (Figures 3/13, 4/13 et 5/13) représentent les teneurs en eau présentant la référence / contrôle (Témoin), les données de l'avant-bras droit (Droite) et les données d'avant-bras gauche (Gauche) du bénévole 03. Tous ces résultats sont extraits à partir de 3 profondeurs fixes savoir à 5, 10 et 15 µm et ont montré :
L'évaluation de l'ASC à 3470 cm⁻¹ indique l'eau non liée. L'ENL est considérée comme l'eau qui n'est pas directement liée aux composants de SC, ne présentant donc pas de liaisons hydrogène avec des lipides ou des protéines du SC. Ceci peut être défini en tant que l'eau présente dans un système de couches (deuxième couche, la troisième couche.) qui peut être trouvé, même à de faibles valeurs d'humidité relative (Vyumvuhore R, Tfayli A, Duplan H, Delalleau A, Manfait M, Baillet-Guffroy A. Analyst 138 (2013) 4103-4111). En comparant les valeurs totales des ASC des formules A et B (Figure 3/13, histogrammes 1) au début de l'étude nous avons trouvé que la peau de l'avant-bras droit (formule A) était 2,7 fois plus hydratée que la peau de l'avant-bras gauche (formule B) alors qu'à la fin de l'étude, nous avons trouvé que la peau de l'avant-bras gauche (formule B) est 1,2 plus hydratée que la peau de l'avant-bras droit (formule A). Nous remarquons que l'hydratation est nettement améliorée à la profondeur de 15 µm: l'hydratation est 3,3 fois supérieure à la fin de l'étude pour la formule B par rapport à la formule de référence A alors qu'une hydratation légèrement supérieure de la formule A (1,2 fois) était observée au début de l'étude.

L'évaluation des ASC à 3280 cm⁻¹ et 3345 cm⁻¹ est indicative de l'eau partiellement liée (EPL). L'eau étroitement liée (EEL) l'est aux sites polaires de protéines du SC constituant une première monocouche de l'eau, alors que l'EPL est liée à la première monocouche d'eau et à d'autres composants moléculaires du SC. Cela signifie que les molécules d'eau de l'EPL interagissent partiellement avec les molécules voisines en utilisant seulement 2 ou 3 des 4 liaisons hydrogène possibles. Ainsi les bandes à 3280 cm⁻¹ et 3345 cm⁻¹ et leurs ASC sont associées avec l'EPL.

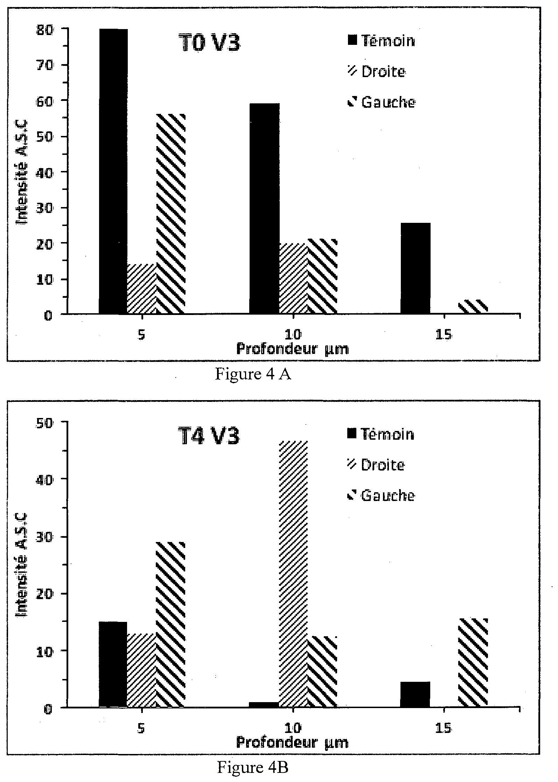

En comparant les valeurs totales des ASC des formule A et B (Figure 4/13, histogrammes 2) au début de l'étude nous trouvions que la peau de l'avant-bras droit (formule B) était 2,6 fois plus hydratée que la peau de l'avant-bras gauche (formule A) et à la fin de l'étude nous avons trouvé que l'hydratation totale était équivalente avec les deux formules. Cependant, nous avons constaté que l'hydratation est nettement améliorée à la profondeur de 15 µm : l'EPL est 16 fois plus importante à la fin de l'étude pour la formule B que pour la formule A de référence.

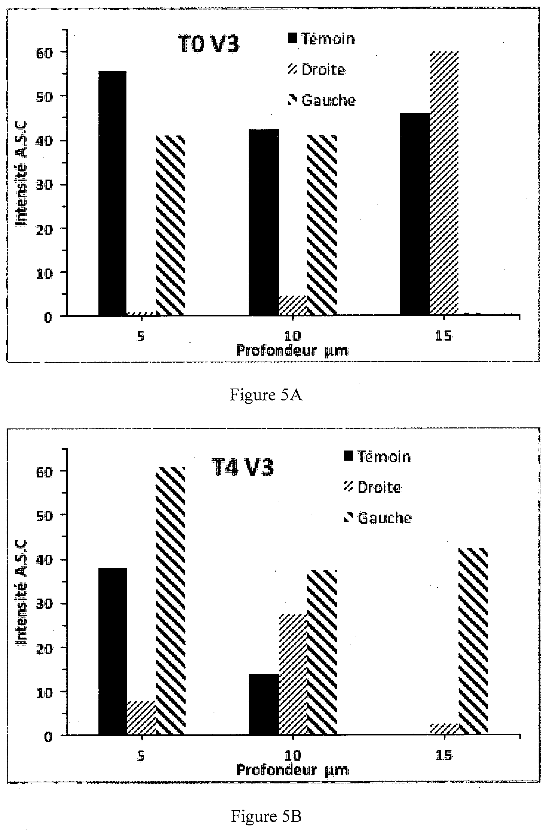

L'évaluation des ASC de la bande à 3210 cm⁻¹ indique l'eau étroitement liée (EEL). En plus de l'étirement NH, les ASC de la bande à 3210 cm⁻¹ peut être liée à l'eau étroitement liée (eau primaire liée). Dans ce cas, les molécules d'eau sont impliquées comme de doubles accepteurs et doubles donneurs de liaison hydrogène donnant une bande vOH autour de 3220 cm⁻¹. En comparant les valeurs totales des ASC de formule A et B (Figure 5/13, histogrammes 3) au début de l'étude nous trouvions que la peau d'avant-bras droit (formule B) contient 1,7 fois plus d'EEL que la peau de l'avant-bras gauche (formule A) et à la fin de l'étude nous trouvons que la peau de l'avant-bras gauche (formule B) contient aussi 3,6 plus d'EEL que la peau de l'avant-bras droit (formule A). Nous remarquons que l'hydratation est nettement améliorée en profondeur de 15 µm : l'EEL est 8,2 fois plus importante à la fin de l'étude pour la formule B que pour la formule A de référence.

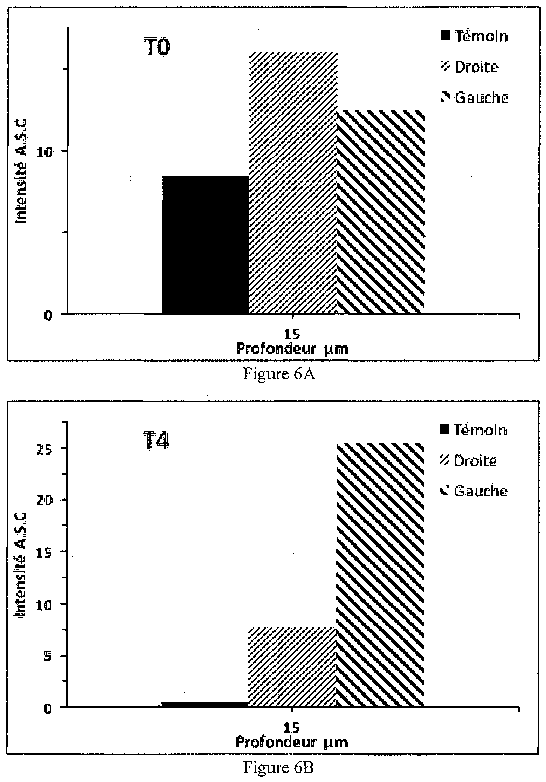

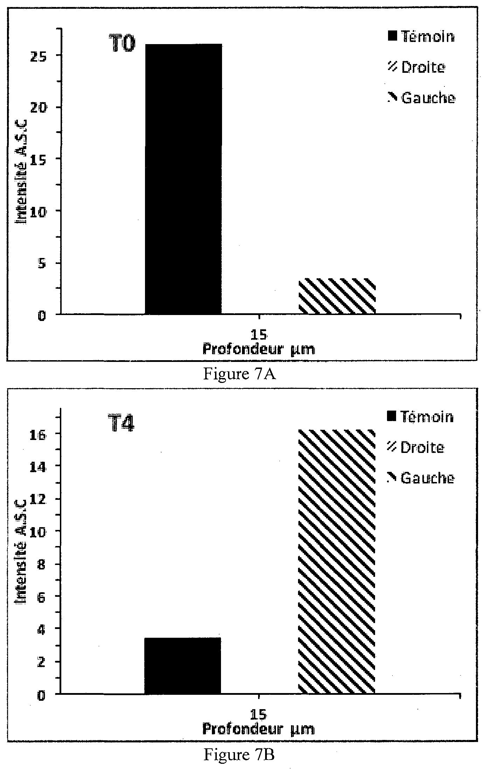

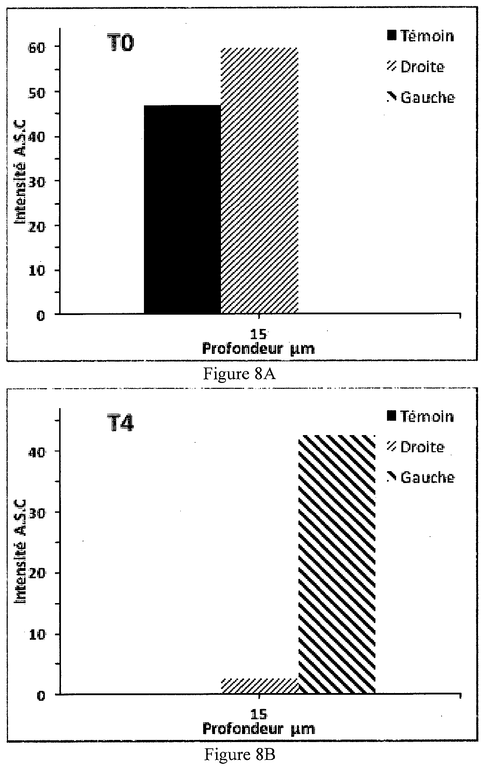

Nous pouvons résumer l'effet de l'hydratation à la profondeur de 15 µm pour les trois types d'eau dans les histogrammes 4 a, b et c (Figures 6/13, 7/13 et 8/13). Nous constatons que l'augmentation d'hydratation pour les trois types d'eau pour la formule B est considérablement augmentée en comparaison avec la formule A.

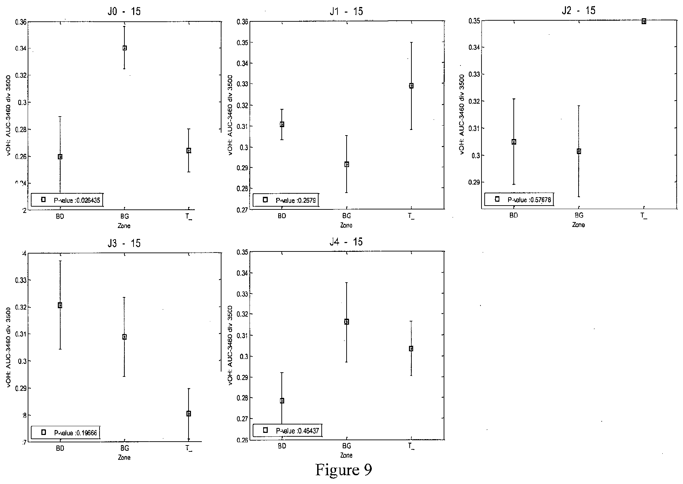

Nous remarquons que l'eau globale qui est une signification de l'hydratation à la profondeur de 15 µm (Figure 9/13, graphique 1) est augmentée pour la peau de l'avant-bras gauche par rapport la peau de l'avant-bras droit en 28 jours d'application.

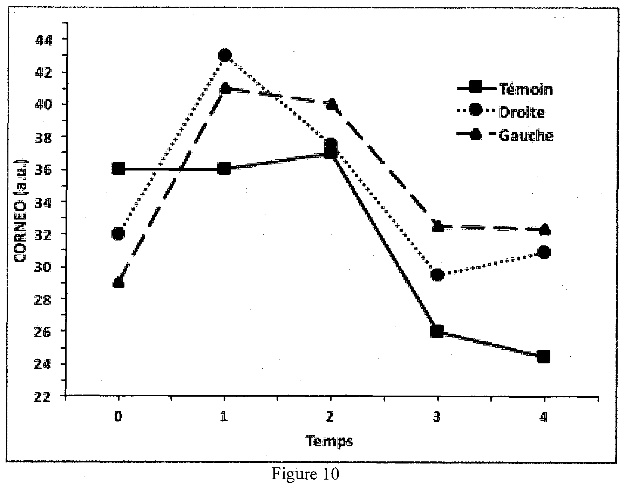

Les résultats de l'étude de cornéométrie (CORNEO) ont montré une nette amélioration de l'hydratation de la peau due à l'application de la formule B par rapport la formule A et le contrôle. En effet, l'amélioration est cumulative ; à la fin de l'étude, à 28 jours, nous avons remarqué une amélioration de l'hydratation par rapport le début de l'étude (Figure 10/13, Graphique 2).

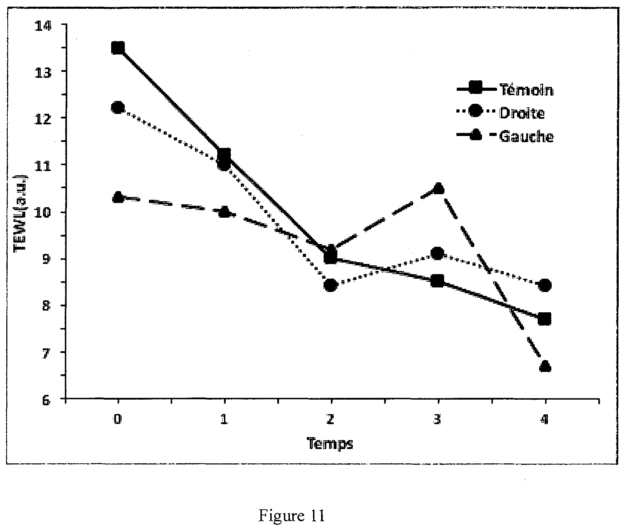

La PIE est la mesure de la perte trans-épidermique en eau ; elle est utilisée pour l'étude de la barrière d'eau et la fonction de la peau humaine. Une mesure basse représente une perte minimale en eau. Plus la mesure est basse, plus la couche protectrice est efficace Ce diagramme met en évidence la supériorité de la Formule B (Figure 11/13 : Graphique 3).

Il est bien connu que l'élasticité et l'efficacité de la fonction barrière du SC sont fortement affectées par son degré d'hydratation [T. Richter, J.H. Müller, U.D. Schwarz, R. Wepf, R. Wiesendanger, App. Phys. A: Mat. Sci. Proc., 72 (2001) S125-S128; R.R. Warner, K.J. Stone, Y.L. Boissy, J Invest Dermatol, 120 (2003) 275-284]. Par conséquent, il est d'un grand intérêt d'examiner l'interaction de l'eau avec les constituants du SC. Les molécules d'eau interagissent avec les groupements polaires des molécules du SC augmentant par la même occasion les espaces intermoléculaires et intramoléculaires. L'interaction eau-lipides, au niveau des têtes polaires probablement, pourrait conduire à une modification de la conformation de chaînes hydrocarbonées et surtout de leur organisation supramoléculaire. Les conformations des lipides du SC sont souvent évaluées en spectroscopie Raman par la comparaison du pic à 1080 cm⁻¹ (témoin des conformations *gauches*) et les pics centrés sur 1060 et 1130 cm⁻¹ (témoins des conformations *trans*). Contrairement aux conformations *gauches,* les conformations *trans* sont associées à un état d'organisation plus compact.

Afin d'étudier les changements biochimiques dans le SC, nous avons concentré notre étude sur des caractéristiques spectrales différentes ; le rapport νₐₛCH₂ (2885 cm⁻¹) / vₛCH₂ (2850 cm⁻¹), généralement utilisé comme un indicateur à la fois de l'état conformationnel et de l'empilement latéral. Des valeurs élevées sont associées à un contenu de conformation *trans* supérieure qui signifie une organisation plus compacte.

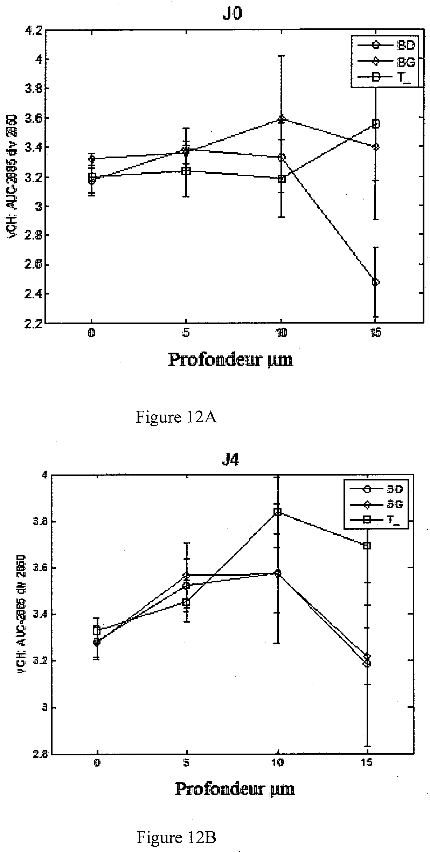

Nous remarquons que le taux de ν_{asym}CH₂ (2885 cm⁻¹) /ν_{sym}CH₂ (2850 cm⁻¹) pour les trois volontaires est légèrement augmenté au dernier jour pour la peau de l'avant-bras gauche (Figure 12/13, graphique 4).

La v (CC) du mode optique dans la région empreinte 1050-1140 cm-¹, liée à la présence de conformation *trans* ou *gauche* à l'intérieur des chaînes acyle. Les ratios de conformation *trans-gauche* ont été analysés par calcul du rapport ASC 1127 + 1081 cm⁻¹ / ASC 1062 cm⁻¹. Des valeurs élevées de ce rapport sont associées à un état d'assemblage compact des lipides alors qu'une diminution est indicative de relâchement.

Nous remarquons que ce rapport est augmenté dès le début de l'étude et jusqu'à la fin, à 28 jours. Cela signifie que les lipides sont plus compacts ce qui correspond à une amélioration de l'hydratation et le relâchement de la peau est diminué (Figure 13/13, graphique 5).

## Revendications

1. Utilisation cosmétique non-thérapeutique pour hydrater la peau d'une composition cosmétique destinée à une application topique, sur la peau, ladite composition comprenant de l'eau de source comprenant :
- du sodium dans une concentration comprise entre 85 mg/L et 150 mg/L, et du potassium dans une concentration comprise entre 35 et 60 mg/L ;
- du calcium, dans une concentration comprise entre 50 et 230 mg/L ;
ladite eau de source ayant une minéralisation comprise entre 650 mg/L et 1100 mg/l, de préférence entre 780 et 1000 mg/L.

2. Utilisation cosmétique non-thérapeutique pour hydrater la peau selon la revendication 1, où ladite eau de source comprend en outre :
• Du magnésium dans une concentration comprise entre 10 et 80 mg/L et/ou
• Du bicarbonate dans une concentration comprise entre 150 et 195 mg/L et/ou
• Du Chlorure dans une concentration comprise entre 160 mg/L et 260 ml/L et/ou
• Des fluorures dans une concentration comprise entre à 0,50 et 0,8 mg/L et/ou
• Du nitrate dans une concentration comprise entre à 25 mg/L et 145 mg/L et/ou
• Du sulfate dans une concentration comprise entre à 120 et 190 mg/L.

3. Utilisation cosmétique non-thérapeutique pour hydrater la peau selon l'une quelconque des revendications précédentes, **caractérisée en ce que** ladite eau de source est présente à hauteur de concentrations allant de 0,01% à environ 90% en poids de la composition, de préférence d'environ 0,1% à environ 75% en poids de la composition, plus préférablement d'environ 1,0% à environ 50% en poids de la composition et plus préférablement encore d'environ 1,0% à environ 25% en poids de la composition.

4. Utilisation cosmétique non-thérapeutique pour hydrater la peau selon l'une quelconque des revendications précédentes, où ladite composition est sous la forme d'une crème blanche ou colorée, d'une pommade, d'un lait, d'une lotion, d'un sérum, d'une pâte, d'une mousse, de gel-crèmes, d'émulsions, de solutions aqueuses, de gels, de pommades, d'aérosols, de stick ou de poudre pressé.

5. Utilisation cosmétique non-thérapeutique pour hydrater la peau selon l'une quelconque des revendications précédentes, où ladite composition comprend l'eau de source ayant une minéralisation comprise entre 650 mg/L et 1100 mg/l, du sodium dans une concentration comprise entre 85 mg/L et 150 mg/L, du potassium dans une concentration comprise entre 35 et 60 mg/L, du calcium, dans une concentration comprise entre 50 et 230 mg/L, du magnésium dans une concentration comprise entre 10 et 80 mg/L, du bicarbonate dans une concentration comprise entre 150 et 195 mg/L du Chlorure dans une concentration comprise entre 160 mg/L et 260 ml/L, des fluorures dans une concentration comprise entre à 0,50 et 0,8 mg/L du nitrate dans une concentration comprise entre à 25 mg/L et 145 mg/L et du sulfate dans une concentration comprise entre à 120 et 190 mg/L, de l'isopropyl palmitate, de l'alcool cétéarylique & Polysorbate 60, de la glycérine, de la gomme xanthane, de l'acétate de tocophérol, et du 5-Chloro-2-méthylisothiazolin-3-one.

## Patentansprüche

1. Nicht-therapeutische kosmetische Verwendung zur Hautbefeuchtung von einer kosmetischen Zusammensetzung, die zur topischen Anwendung auf der Haut bestimmt ist, wobei die Zusammensetzung Quellwasser umfasst, umfassend:
- Natrium in einer Konzentration zwischen 85 mg/l und 150 mg/l und Kalium in einer Konzentration zwischen 35 und 60 mg/l;
- Calcium in einer Konzentration zwischen 50 und 230 mg/l;
wobei das Quellwasser eine Mineralisierung zwischen 650 mg/l und 1100 mg/l, vorzugsweise zwischen 780 und 1000 mg/l, aufweist.

2. Nicht-therapeutische kosmetische Verwendung zur Hautbefeuchtung nach Anspruch 1, wobei das Quellwasser ferner umfasst:
• Magnesium in einer Konzentration zwischen 10 und 80 mg/l, und/oder
• Bicarbonat in einer Konzentration zwischen 150 und 195 mg/l, und/oder
• Chlorid in einer Konzentration zwischen 160 mg/l und 260 ml/l; und/oder
• Fluoride in einer Konzentration zwischen 0,50 und 0,8 mg/l, und/oder
• Nitrat in einer Konzentration zwischen 25 mg/l und 145 mg/l, und/oder
• Sulfat in einer Konzentration zwischen 120 und 190 mg/l.

3. Nicht-therapeutische kosmetische Verwendung zur Hautbefeuchtung nach einem der vorstehendenAnsprüche, **dadurch gekennzeichnet, dass** das Quellwasser in Konzentrationshöhen von 0,01 Gew.-% bis etwa 90 Gew.-% der Zusammensetzung, vorzugsweise von etwa 0,1 Gew.-% bis etwa 75 Gew.-% der Zusammensetzung, noch bevorzugter von etwa 1,0 Gew.-% bis etwa 50 Gew.-% der Zusammensetzung, und am meisten bevorzugt von etwa 1,0 Gew.-% bis etwa 25 Gew.-% der Zusammensetzung vorliegt.

4. Nicht-therapeutische kosmetische Verwendung zur Hautbefeuchtung nach einem der vorstehenden Ansprüche, wobei die Zusammensetzung in Form einer weißen oder farbigen Creme, einer Salbe, einer Milch, einer Lotion, eines Serums, einer Paste, eines Schaums, von Gel-Cremes, Emulsionen, wässrigen Lösungen, Gelen, Aerosolen, eines Stifts oder eines gepressten Pulvers vorliegt.

5. Nicht-therapeutische kosmetische Verwendung zur Hautbefeuchtung nach einem der vorstehenden Ansprüche, wobei die Zusammensetzung Quellwasser mit einer Mineralisierung zwischen 650 mg/l und 1100 mg/l, Natrium in einer Konzentration zwischen 85 mg/l und 150 mg/l, Kalium in einer Konzentration zwischen 35 und 60 mg/l, Calcium in einer Konzentration zwischen 50 und 230 mg/l, Magnesium in einer Konzentration zwischen 10 und 80 mg/l, Bicarbonat in einer Konzentration zwischen 150 und 195 mg/l, Chlorid in einer Konzentration zwischen 160 mg/l und 260 mg/l, Fluoride in einer Konzentration zwischen 0,50 und 0,8 mg/l, Nitrat in einer Konzentration zwischen 25 mg/l und 145 mg/l und Sulfat in einer Konzentration zwischen 120 und 190 mg/l, Isopropylpalmitat, Cetearylalkohol und Polysorbat 60, Glycerin, Xanthangummi, Tocopherolacetat und 5-Chlor-2-methylisothiazolin-3-on umfasst.

## Claims

1. Non-therapeutic cosmetic use for moisturizing the skin of a cosmetic composition intended for topical application to the skin, said composition comprising spring water comprising:
- sodium in a concentration between 85 mg/L and 150 mg/L, and potassium in a concentration between 35 and 60 mg/L;
- calcium, in a concentration between 50 and 230 mg/L;
said spring water having a mineralization between 650 mg/L and 1100 mg/l, preferably between 780 and 1000 mg/L.

2. Non-therapeutic cosmetic use for moisturizing the skin according to claim 1, wherein said spring water further comprises:
• magnesium in a concentration between 10 and 80 mg/L and/or
• bicarbonate in a concentration between 150 and 195 mg/L and/or
• chloride in a concentration between 160 mg/L and 260 ml/L and/or
• fluorides in a concentration between 0.50 and 0.8 mg/L and/or
• nitrate in a concentration between 25 mg/L and 145 mg/L and/or
• sulfate in a concentration between 120 and 190 mg/L.

3. Non-therapeutic cosmetic use for moisturizing the skin according to either of the preceding claims, **characterized in that** said spring water is present in concentrations ranging from 0.01% to about 90% by weight of the composition, preferably from about 0.1% to about 75% by weight of the composition, more preferably from about 1.0% to about 50% by weight of the composition and still more preferably from about 1.0% to about 25% by weight of the composition.

4. Non-therapeutic cosmetic use for moisturizing the skin according to any of the preceding claims, wherein said composition is in the form of a white or colored cream, ointment, milk, lotion, serum, paste, foam, gel-creams, emulsions, aqueous solutions, gels, ointments, aerosols, stick or pressed powder.

5. Non-therapeutic cosmetic use for moisturizing the skin according to any of the preceding claims, wherein said composition comprises spring water having a mineralization between 650 mg/L and 1100 mg/l, sodium in a concentration between 85 mg/L and 150 mg/L, potassium in a concentration between 35 and 60 mg/L, calcium in a concentration between 50 and 230 mg/L, magnesium in a concentration between 10 and 80 mg/L, bicarbonate in a concentration between 150 and 195 mg/L, chloride in a concentration between 160 mg/L and 260 ml/L, fluorides in a concentration between 0.50 and 0.8 mg/L, nitrate in a concentration between 25 mg/L and 145 mg/L and sulfate in a concentration between 120 and 190 mg/L, isopropyl palmitate, cetearyl alcohol & Polysorbate 60, glycerin, xanthan gum, tocopherol acetate and 5-Chloro-2-methylisothiazolin-3-one.
